# EUROPEAN PATENT APPLICATION

(11) **EP 4 556 454 A1**
(43) Date of publication of application: **21.05.2025**
(21) Application number: 22951177.9
(22) Date of filing: 15.07.2022
(51) Int. Cl.: C07C 1/04, C07C 9/04

(54) **METHANE SYNTHESIS SYSTEM**

(71) Applicant: MITSUBISHI ELECTRIC CORPORATION, Chiyoda-ku Tokyo 100-8310 (JP)
(72) Inventor: SHINOKI, Toshio, Tokyo 100-8310 (JP); ONAKA, Yoji, Tokyo 100-8310 (JP); NAKASHIMA, Seiji, Tokyo 100-8310 (JP); KAWAMOTO, Makoto, Tokyo 100-8310 (JP); TANISHIMA, Makoto, Tokyo 100-8310 (JP)
(74) Representative: Meissner Bolte Partnerschaft mbB
(86) International application number: PCT/JP2022/027806
(87) International publication number: WO 2024/013968

(57) **Abstract**

A methane synthesis system according to the present invention includes: a co-electrolysis part that obtains hydrogen and carbon monoxide by electrolyzing water and carbon dioxide, a methanation reaction part that obtains a product gas containing methane by a methanation reaction that uses the hydrogen and the carbon monoxide, and a cooler having a distribution channel in which a refrigerant capable of phase transition, is distributed. The cooler cools the methanation reaction part using heat of vaporization from vaporizing at least a portion of the refrigerant on an inside of the distribution channel.

## Description

### Technical Field

The present invention relates to a methane synthesis system.

### Background Art

Patent Document 1 discloses a production system that produces hydrocarbons using carbon dioxide and water. Said production system reduces water and carbon dioxide to obtain a mixed gas that contains hydrogen and carbon monoxide. Said production system generates hydrocarbons such as methane or the like, using the mixed gas.

### Citation List

### Patent Documents

Patent Document 1: PCT International Publication No. WO 2021/201192 A1

### Summary of the Invention

### Problem to be Solved by the Invention

In the aforementioned production system, a risk of reduction in methane gas generation rate exists.

The present invention has been made in order to address the problem above, and an object is to provide a methane synthesis system where it is possible to increase a production efficiency of methane.

### Means to Solve the Problem

A methane synthesis system according to one embodiment of the present invention includes: a co-electrolysis part that obtains hydrogen and carbon monoxide by electrolyzing water and carbon dioxide, a methanation reaction part that obtains a product gas containing methane by a methanation reaction that uses the hydrogen and the carbon monoxide, and a cooler having a distribution channel in which a refrigerant capable of phase transition, is distributed. The cooler cools the methanation reaction part using heat of vaporization from vaporizing at least a portion of the refrigerant on an inside of the distribution channel.

### Effects of the Invention

According to the present invention, it is possible to provide a methane synthesis system where it is possible to increase a production efficiency of methane.

### Brief Description of the Drawings

FIG. 1 A schematic drawing showing a methane synthesis system according to a first embodiment.
FIG. 2 A schematic drawing showing a methane synthesis system according to a second embodiment.
FIG. 3 A schematic drawing showing a methane synthesis system according to a third embodiment.

### Description of Embodiments

Hereinafter, embodiments of the present invention are explained with reference to the drawings. The scope of the present invention is not limited to the embodiments below, and embodiments may be changed so long as the embodiments do not depart from the technical scope of the present invention.

### First Embodiment

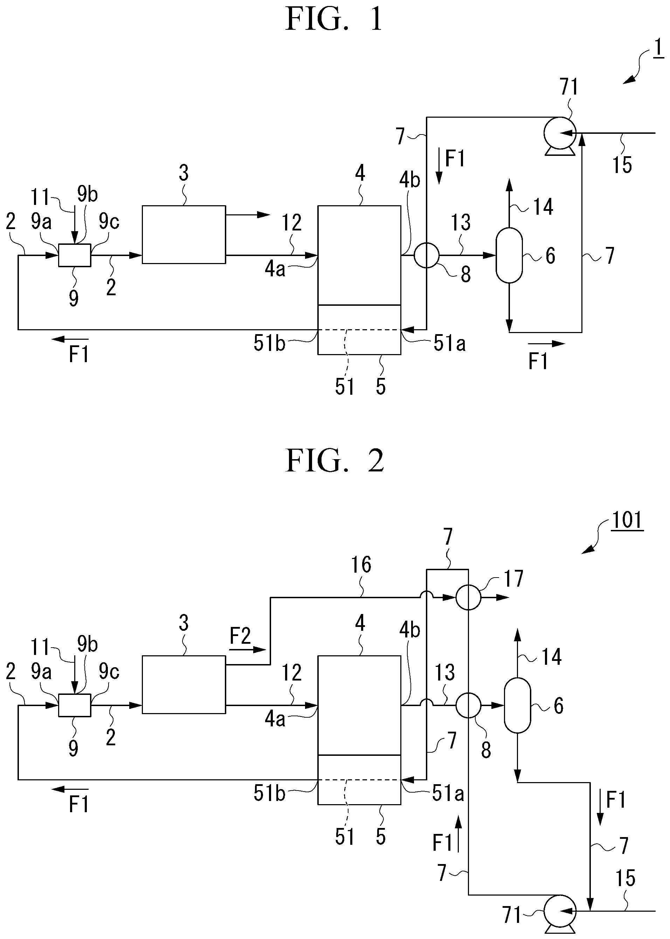

FIG. 1 is a schematic drawing of a methane synthesis system in a first embodiment.

As shown in FIG. 1, a methane synthesis system 1 includes a supply route 2, a co-electrolysis part 3, a methanation reaction part 4, a cooler 5, a separator 6, a capture route 7, a first heat exchanger 8, and an ejector 9.

The supply route 2 guides water (for example, water vapor) and carbon dioxide to the co-electrolysis part 3. The water (for example, water vapor) is supplied from the cooler 5. The carbon dioxide is supplied from an introduction route 11. The supply route 2 for example, guides a mixed gas of the water and the carbon dioxide to the co-electrolysis part 3.

The carbon dioxide that is supplied from the introduction route 11 may be carbon dioxide that is captured from the atmosphere using DAC (Direct Air Capture). The carbon dioxide that is supplied from the introduction route 11 may be carbon dioxide that is discharged from a solid oxide fuel cell (SOFC: Solid Oxide Fuel Cell).

The co-electrolysis part 3 for example, may be a solid oxide electrolysis cell (SOEC: Solid Oxide Electrolysis Cell), which includes a cathode electrode and an anode electrode. As the solid oxide electrolysis cell for example, solid oxides that have oxygen ion conductivity are used. As an electrolyte, zirconia-based oxides or the like are used. The co-electrolysis part 3 is an example of electrolysis equipment.

The co-electrolysis part 3 supplies the water and the carbon dioxide, which are supplied from the supply route 2, to the cathode electrode of the solid oxide electrolysis cell. It is preferable that the water, which is used for co-electrolysis in the solid oxide electrolysis cell, bej water vapor.

The co-electrolysis part 3 may include a heating device that heats the solid oxide electrolysis cell. The heating device is able to adjust a temperature of the solid oxide electrolysis cell to a temperature that is suitable for the co-electrolysis reaction. A percentage of the carbon dioxide and the water that are supplied to the solid oxide electrolysis cell correspond to the percentage of contents (carbon monoxide, hydrogen) of the target mixed gas.

The co-electrolysis part 3 obtains a mixed gas (mixed fluid) that contains hydrogen (H₂) and carbon monoxide (CO) from water (H₂O) and carbon dioxide (CO₂), using co-electrolysis. Co-electrolysis for example, progresses according to equation (I) shown below. Such reaction is endothermic. The co-electrolysis is an electrolysis reaction that conducts electrolysis of the water and electrolysis of the carbon dioxide simultaneously.

3H₂O + CO₂→CO + 3H₂ + 2O₂ ... (I)

It is possible for the co-electrolysis part 3 for example, to use reusable energy (for example, solar electric power, wind electric power or the like) to generate electricity used to conduct co-electrolysis. Methane obtained by using reusable energy does not generate added carbon dioxide when used in combustion processes, so it is safe to consider the obtained methane as a carbon neutral fuel that does not have an effect on global warming.

The mixed gas obtained at the co-electrolysis part 3 not only has hydrogen (H₂) and carbon monoxide, but also contains water and carbon dioxide that did not react. The mixed gas is guided to the methanation reaction part 4, passing through an extraction route 12.

From the hydrogen (H₂) and the carbon monoxide (CO), the methanation reaction part 4 obtains a product gas (product fluid) that contains water (H₂O) and methane (CH₄), using methanation reaction. The methanation reaction for example, progresses according to equation (II) shown below. Such reaction is an exothermic reaction.

3H₂ + CO→H₂O + CH₄ ... (II)

It is preferable that the methanation reaction part 4 include a methanation catalyst that catalyzes the mixed gas. As a methanation catalyst, an Ni catalyst, an Ru catalyst or the like may be mentioned. The methanation catalyst promotes the methanation reaction.

There are cases where the product gas obtained at the methanation reaction part 4 contains not only water and methane, but contains unreacted hydrogen (H₂), carbon monoxide, and carbon dioxide or the like as well. The product gas is guided to the separator 6, through a discharge route 13.

An inlet 4a of the methanation reaction part 4 is a location where the extraction route 12 connects to the methanation reaction part 4. An outlet 4b of the methanation reaction part 4 is a location where the discharge route 13 connects to the methanation reaction part 4.

The cooler 5 is thermally connected to the methanation reaction part 4. The cooler 5 for example, is connected to the methanation reaction part 4. The cooler 5 for example, is integrally formed with the methanation reaction part 4. Heat transfer between the cooler 5 and the methanation reaction part 4 is possible.

The cooler 5 cools the methanation reaction part 4. A distribution channel 51 in which a refrigerant flows and is distributed, is formed in the cooler 5. A flow direction (a direction from the inlet 51a towards the outlet 51b) in the distribution channel 51 for example, is a direction that is opposite to a flow direction (a direction towards the outlet 4b, from the inlet 4a) in the methanation reaction part 4.

The separator 6 separates a fluid that contains methane, and a fluid that contains water, from the product gas.

Separation methods such as liquefied separation, membrane separation, or absorbent separation for example, are adopted as separation methods in the separator 6. One of the aforementioned separation methods, or a combination of two or more, may be adopted in the separator 6.

The separator 6 that uses liquefied separation for example, separates particular components from other components (gas) by liquefying. Particularly for example, components that include water are liquefied using temperature adjustment, and are separated from the other components (gas) that include methane.

The separator 6 that uses membrane separation for example, separates particular components from other components by allowing components having small particle sizes to permeate a separation membrane. Specifically for example, a separation membrane that selectively allows water to permeate is used. Such separation membrane separates the components that include water, and the components that include methane from the mixed gas.

The separator 6 that uses absorbent separation for example, separates by having an absorbent absorb particular components. As an absorbent, silica gel, zeolite, and activated carbon or the like may be mentioned. Specifically, by having the absorbent absorb that components that include water, it is possible to separate such components from other the components that include methane.

The separator 6 that uses absorbent separation has a function of separating the absorbed components from the absorbent. The absorbent 6 for example, includes a heating device. The heating device separates the absorbed components from the absorbent by heating the absorbent. The separator 6 may include pressure reduction devices such as a pressure reduction pump or the like. The pressure reduction devices promote separation of the absorbed components from the absorbent by having the absorbent remain under reduced pressure.

The components that include methane are extracted from the separator 6, passing through an extraction route 14. The components that include methane for example, are sent as raw materials for city gas or the like, or sent to gas manufacturing equipment and so on.

The capture route 7 is connected to the separator 6 and the cooler 5. The component that contains water (fluid F1 that contains water) is extracted from separator 6, passing through the capture route 7, and is guided to the distribution channel 51 of the cooler 5. A pump 71 that sends the fluid F1 to the cooler 5 is provided in the capture route 7.

A main component of the fluid F1 is water. The fluid F1 is interchangeable between a liquid and a gas. The fluid F1 may include components other than water.

A water supply route 15 is connected to the capture route 7. Water is supplied to the capture route 7 as needed from an outside thereof, using the water supply route 15.

The first heat exchanger 8 is provided on the capture route 7. The first heat exchanger 8 preheats the fluid F1 that flows in the capture route 7, by conducting heat exchange with the product gas that flows in the discharge route 13.

It is possible to use a well-known heat exchanger as the first heat exchanger 8. As the first heat exchanger 8 for example, it is possible to use a multi-tube type heat exchanger, a plate type heat exchanger, a coil type heat exchanger, a double pipe type heat exchanger, and a spiral type heat exchanger or the like.

The fluid F1 is introduced to the distribution channel 51 of the cooler 5, and distributes through the distribution channel 51 as the refrigerant. The methanation reaction part 4 is cooled by exchanging heat with the fluid F1.

At least a portion of the fluid F1 in the inlet 51a of the distribution channel 51 is liquid. At least a portion thereof in a step where the fluid F1 flows through the distribution channel 51 from the inlet 51a towards the outlet 51b is a vaporized. When vaporizing the fluid F1 is, the methanation reaction part 4 is cooled using heat of vaporization.

The ejector 9 is provided on the supply route 2. The ejector 9 has a flow inlet 9a, an intake 9b, and a flow outlet 9c. The fluid F1 that flows in the supply route 2 flows into the ejector 9 from the flow inlet 9a, and flows out from the flow outlet 9c. The fluid F1 is a working fluid. A nozzle that ejects the working fluid is provided on an inside of the ejector 9. The introduction route 11 is connected to the intake 9b. Carbon dioxide flows to the ejector 9 as an intake fluid from the intake 9b, passing through the introduction route 11.

Next, a methane synthesis method employed by the methane synthesis system 1 is explained.

The methane synthesis method according to the present embodiment has a supply step, an electrolysis step, a methanation step, a separation step, and a cooling step.

In the supply step, water (H₂O) and carbon dioxide (CO₂) are guided to the co-electrolysis part 3 using the supply route 2.
In the electrolysis step, the mixed gas that contains hydrogen (H₂) and carbon monoxide (CO) is obtained using co-electrolysis in the co-electrolysis part 3, from the water and carbon dioxide.

In the methanation step, the product gas containing water and methane gas is obtained using the methanation reaction in the methanation reaction part 4. The product gas not only contains water and methane, but also contains carbon monoxide and hydrogen (H₂) that did not react. The product gas is guided to the separator 6, passing through the discharge route 13.

In the separation step, a fluid that contains methane, and a fluid that water, are separated from the product gas in the separator 6.

In the cooling step, the fluid F1 that contains water is extracted from the separator 6, is guided to the distribution channel 51 of the cooler 5 using the capture route 7. At least a portion thereof in a step where the fluid F1 flows through the distribution channel 51 from the inlet 51a towards the outlet 51b is a vaporized. When vaporizing the fluid F1, the methanation reaction part 4 is cooled using the heat of vaporization.

The cooler 5 forms a temperature distribution having a first region, a second region, and a third region, in such order, from the inlet 51a of the distribution channel 51 towards the outlet 51b. The first region is a region where a temperature of the fluid F1 rises. The second region is a region where the fluid F1 vaporizes, while the temperature thereof remains approximately constant. The third region is a region where the temperature of the vaporized fluid F1 rises again.

The temperature of the fluid F1 at the inlet 51a of the distribution channel 51 is lower than the temperature of the fluid F1 at the outlet 51b. The temperature of the fluid F1 at the inlet 51a is for example, 200 °C to 400 °C. The temperature of the fluid F1 at the outlet 51b is for example 450 °C to 650 °C.

The temperature of the methanation reaction part 4 is a temperature that corresponds to the cooler 5. In other words, a temperature at the outlet 4b is lower than temperature at the inlet 4a. A temperature of an inside of the methanation reaction part 4 at the outlet 4b is for example, 200 °C to 400 °C. A temperature of the inside of the methanation reaction part 4 at the inlet 4a is for example, 450 °C to 650 °C.

The fluid F1 that contains water (water vapor), is introduced along with the carbon dioxide that is introduced by the ejector 9, to the co-electrolysis part 3, passing through the supply route 2.

The cooler 5 cools the methanation reaction part 4 using the heat of vaporization that results from vaporizing at least a portion of the fluid F1, which is the refrigerant, in the distribution channel 51, in the methane synthesis system 1. For example, the cooler 5 has the first region in which the temperature of the fluid F1 rises, the second region where the fluid F1 is vaporized while the temperature thereof remains approximately constant, and the third region where the temperature of the fluid F1 rises again.

In the methanation reaction part 4, by having heat of reaction of the methanation reaction be suitably taken away at the second region, it is possible to optimize the temperature of the inside of the methanation reaction part 4. Therefore, it is possible to have the methanation reaction proceed efficiently. Thus, it is possible to increase the production efficiency of methane.

Since the methane synthesis system 1 uses the fluid F1 that contains water as a refrigerant, it is possible for the methane synthesis system 1 to furnish a suitable temperature distribution to the methanation reaction part 4. Thus, it is possible to have the methanation reaction in the methanation reaction part 4 proceed efficiently.

Since the methane synthesis system 1 includes the supply route 2 that guides the fluid F1, which passes through the cooler 5, to the co-electrolysis part 3, it is possible to efficiently utilize heat obtained at the cooler 5 by the fluid F1, at the co-electrolysis part 3. Thus, it is possible to increase the energy efficiency of the entire system.

Since the methane synthesis system 1 includes the ejector 9, for example, it is possible to have more energy savings when compared to a case where only the blower is used to guide the carbon dioxide to the supply route 2.

Since the methane synthesis system 1 includes the separator 6, which separates the fluid F1 that contains water from the product gas of the methanation reaction, and the capture route 7 that guides the fluid F1 to the cooler 5, it is possible to effectively utilize the water that is a by-product of the methanation reaction, and it is possible to increase the efficiency of the methane synthesis system.

### Second Embodiment

Next, a methane synthesis system according to a second embodiment is explained. Since the methane synthesis system according to the second embodiment of the present invention is fundamentally the same as the methane synthesis system according to the first embodiment, mainly aspects in which the systems differ are explained. Configurations which are similar across other embodiments have the same reference signs affixed thereto, with explanations thereof being omitted.

FIG. 2 is a schematic drawing showing a methane synthesis system according to a second embodiment.

As shown in FIG. 2, a methane synthesis system 101 differs from the methane synthesis system 1 (refer to FIG. 1) in an aspect of including an extraction route 16, and a second heat exchanger 17. The second heat exchanger 17 is an example of a "heat exchanger".

The co-electrolysis part 3 generates oxygen (O₂) at the anode, by electrolyzing the water and the carbon dioxide previously mentioned.

The extraction route 16 extracts a fluid F2 that contains oxygen (O₂) that is produced at the co-electrolysis part 3.

The second heat exchanger 17 is provided on the capture route 7. The second heat exchanger 17 heats the fluid F1 that flows in the capture route 7 by conducting heat exchange with the fluid F2 that is extracted by the extraction route 16.

It is possible to use a well-known heat exchanger as the second heat exchanger 17. As the second heat exchanger 17, it is possible to use a multi-tube type heat exchanger, a plate type heat exchanger, a coil type heat exchanger, a double pipe type heat exchanger, and a spiral type heat exchanger or the like.

As with the methane synthesis system 1 (refer to FIG. 1), since it is possible for the methane synthesis system 101 to optimize the temperature of the inside of the methanation reaction part 4, it is possible to increase a production efficiency of methane. Besides the above, the methane synthesis system 101 achieves the same effects as the methane synthesis system 1 (refer to FIG. 1).

It is possible for the methane synthesis system 101 to preheat the fluid F1, using the second heat exchanger 17. As such, it is possible to effectively use heat of the fluid F2, and it is possible to increase the energy efficiency of the entire system.

### Third Embodiment

A methane synthesis system according to a third embodiment is explained. Configurations which are similar across other embodiments have the same reference signs affixed thereto, with explanations thereof being omitted.

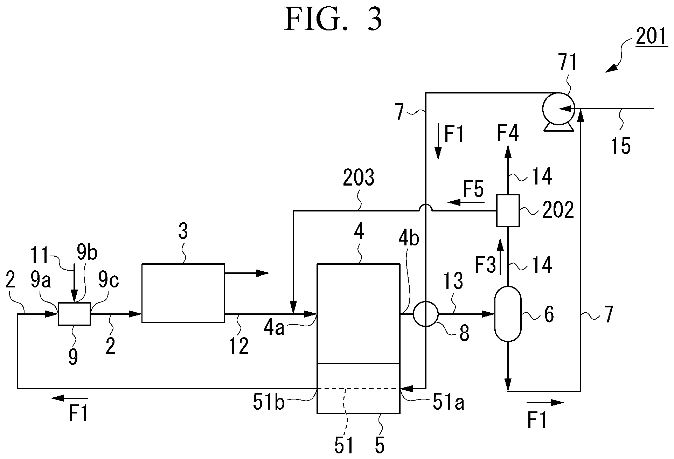

FIG. 3 is a schematic drawing showing a methane synthesis system according to a third embodiment.

As shown in FIG. 3, a methane synthesis system 201 differs from the methane synthesis system 1 (refer to FIG. 1) in an aspect of including a methane purifier 202, and a return route 203. The methane purifier 202 is provided on the extraction route 14. The extraction route 14 is a route that extracts components which contain methane (a fluid F3 that contains methane) from the separator 6. The methane purifier 202 purifies the methane contained in the fluid F3, and obtains a fluid F4 having a high concentration of methane. The purifier 202 is an example of a "methane purifying part".

It is possible to adopt purifying methods in the methane purifier 202 such as membrane separation, liquefied separation, absorption separation or the like, with membrane separation being the most preferable method. The methane purifier 202 that uses membrane separation for example, includes a separation membrane that allows methane to permeate.

The return route 203 connects an outlet of a non-permeable side of the methane purifier 202 and the extraction route 12. The return route 203 returns a fluid F5 that did not permeate the separation membrane in the methane purifier 202, to the methanation reaction part 4 via the extraction route 12. The return route 203 may be a route that connects the outlet of the non-permeable side of the methane purifier 202 and the methanation reaction part 4.

The fluid F3 that is extracted using the extraction route 14 from the separator 6 is guided to the methane purifier 202, in the methane synthesis system 201. The fluid F4 of a permeable side having an increased concentration of methane in the methane purifier 202 is guided to the outside of the system as a purified product gas (purified product fluid). A step in which the methane purifier 202 is used to purify the methane contained in the fluid F3 is referred to as a "purification step".

The fluid F5 that does not permeate the separation membrane of the methane purifier 202 is returned to the extraction route 12, using the return route 203. The fluid F5 is then introduced into to the methanation reaction part 4, from the extraction route 12. As such, it is possible to facilitate methanation of unreacted byproducts (for example, hydrogen, and carbon monoxide) contained in the fluid F5. Thus, it is possible to increase the production efficiency of methane at the methanation reaction part 4.

The liquid F5 on the non-permeable side is a residual gas (residual fluid) obtained by separation of the fluid F3 from the fluid F4 of the permeable side.

As with the methane synthesis system 1 (refer to FIG. 1), since it is possible for the methane synthesis system 201 to optimize the temperature of the inside of the methanation reaction part 4, it is possible to increase the production efficiency of methane. Besides the above, the methane synthesis system 201 achieves the same effects as the methane synthesis system 1 (refer to FIG. 1).

Since the methane synthesis system 201 includes the methane purifier 202, it is possible to obtain the fluid F4 (purified product gas) having a high concentration of methane.

Since the methane synthesis system 201 has the return route 203, it is possible to return the fluid F5 (residual gas) of the non-permeable side to the methanation reaction part 4. Thus, it is possible to increase a production efficiency of methane at the methanation reaction part 4.

The present invention is not limited to the various configurations and various methods explained above, and changes may be added as needed, so long as no conflicts in the technical scope thereof occurs.

For example, in the first embodiment, although an example where the co-electrolysis part 3 uses a solid oxide electrolysis cell (SOEC) is shown, the co-electrolysis part may adopt a different method. The co-electrolysis part for example, may be a solid polymer type co-electrolysis part.

In the first embodiment, although the co-electrolysis part 3 that obtains hydrogen and carbon monoxide from water and carbon dioxide is used, a device for obtaining hydrogen (H₂) and carbon monoxide is not limited to the co-electrolysis part. For example, it is possible to use an electrolysis device that independently conducts a step for obtaining carbon monoxide by electrolyzing carbon dioxide, and a step for obtaining hydrogen (H₂) by electrolyzing water.

### List of Reference Signs

- 1,101,201: Methane Synthesis System
- 2: Supply Route
- 3: Co-electrolysis Part
- 4: Methanation reaction Part
- 4a: Inlet
- 4b: Outlet
- 5: Cooler
- 6: Separator
- 7: Capture Route
- 9: Ejector
- 17: Second Heat Exchanger (Heat Exchanger)
- 202: Methane Purifier (Methane Purifying Part)
- 203: Return Route

## Claims

1. A methane synthesis system comprising:
- a co-electrolysis part that obtains hydrogen and carbon monoxide by electrolyzing water and carbon dioxide;
- a methanation reaction part that obtains a product gas containing methane by a methanation reaction that uses the hydrogen and the carbon monoxide; and
- a cooler having a distribution channel in which a refrigerant capable of phase transition, is distributed,
wherein
the cooler cools the methanation reaction part using heat of vaporization from vaporizing at least a portion of the refrigerant on an inside of the distribution channel, and lowers a temperature at an outlet of the methanation reaction part, more than a temperature at an inlet thereof.

2. The methane synthesis system according to claim 1,
wherein the refrigerant is a fluid that contains water.

3. The methane synthesis system according to claim 2
further comprising:
- a supply route that guides the refrigerant as the water, which passes through the cooler, to the co-electrolysis part.

4. The methane synthesis system according to claim 3,
wherein an ejector that takes in the carbon dioxide, with the water as a working fluid, is provided on the supply route.

5. The methane synthesis system according to any one of claims 2 to 4 further comprising:
- a separator that separates water from the product gas, and
- a capture route that guides the water separated at the separator as the refrigerant to the cooler.

6. The methane synthesis system according to claim 5,
wherein a heat exchanger is provided on the capture route, and heats the water by conducting heat exchange with oxygen produced by co-electrolysis of the water and the carbon dioxide at the co-electrolysis part.

7. The methane synthesis system according to any one of claims 1 to 4 further comprising:
- a separator that separates a fluid that contains the methane from the product gas, and
- a methane purifier that purifies the methane contained in the fluid, and obtains a purified product gas.

8. The methane synthesis system according to claim 7 further comprising:
a return route that returns a residual gas obtained from separating the purified product gas from the fluid that contains the methane, to the methanation reaction part.
